# EUROPÄISCHE PATENTSCHRIFT

(11) **EP 1 981 550 B1**
(45) Veröffentlichungstag und Bekanntmachung des Hinweises auf die Patenterteilung: **13.07.2016**
(21) Anmeldenummer: 06806592.9
(22) Anmeldetag: 27.10.2006
(51) Int. Cl.: A61L 15/26, B32B 5/32, B32B 27/06, B32B 27/40, B29C 47/02, B29C 47/88

(54) **WUNDABDECKUNG UND DESSEN EXTRUSIONSBESCHICHTUNGSHERSTELLUNGSVERFAHREN**
WOUND COVERING AND ITS METHOD OF PRODUCTION BY EXTRUSION COATING
PANSEMENT POUR PLAIES ET SON PROCÉDÉ DE FABRICATION PAR REVÊTEMENT PAR EXTRUSION

(30) Priorität: 06.02.2006 EP 06101315
(43) Veröffentlichungstag der Anmeldung: 22.10.2008
(73) Patentinhaber: nolax AG, 6203 Sempach Station (CH)
(72) Erfinder: LEUMANN, Manuel, 5712 Beinwil am See (CH); DOBMANN, Andreas, 6208 Oberkirch (CH)
(74) Vertreter: Hepp Wenger Ryffel AG
(86) Internationale Anmeldenummer: PCT/EP2006/010382
(87) Internationale Veröffentlichungsnummer: WO 2007/090444

(56) Entgegenhaltungen:
- EP-A- 0 651 984
- WO-A-91/01706
- WO-A-95/15135
- DE-A1- 3 515 580
- US-A- 3 668 050
- US-A- 4 854 995
- US-A- 5 147 338
- US-A- 5 753 342
- US-A- 6 165 625
- STOKES VK: "Joining Methods for Plastics and Plastic Composites" POLYMER ENGINEERING AND SCIENCE, Bd. 29, Nr. 19, Oktober 1989 (1989-10), Seiten 1310-1324, XP002418754

## Beschreibung

Die Erfindung betrifft das Gebiet der auf Schaumstoffmaterialien basierenden Wundabdeckungen, insbesondere Herstellungsverfahren für solche Wundabdeckungen.

Wundabdeckungen sollen eine Vielzahl von Funktionen erfüllen: So z.B. die Gewährleistung der Keimdichtigkeit und die Etablierung eines physiologischen Heilmilieus, wobei hierbei heute bei sekundär heilenden Wunden ein feuchtes Heilmilieu bevorzugt ist. Weiter sollte die Wundabdeckung keine Zytotoxizität oder Allergenität aufweisen und atraumatisch entfernbar/auswechselbar sein. Ferner muss überschüssiges Wundexsudat von der Wunde abgeleitet werden.

In der Wundversorgung finden heute vermehrt Schaumstoff-basierte Wundabdeckungen Verwendung, insbesondere für chronische Wunden. Derartige Schaumstoff-Wundabdeckungen haben den Vorteil, dass Wundexsudat zuverlässig von der Wunde entfernt werden kann aufgrund der Saugfähigkeit des Schaumstoffs. Gegenüber altbekannten Wundabdeckungen wie bspw. Gaze, Mullbinden etc. bieten Schaumstoff-basierte Wundabeckungen also den Vorteil einer grösseren Aufnahmekapazität für Wundexsudat, sowie den weiteren Vorteil, dass kein Verkleben mit der Wunde eintritt und somit ein schmerzfreier Verbandwechsel möglich ist.

Auf der wundabgewandten Seite sind diese Schaumstoff-Wundabdeckungen in der Regel mit einer Keimbarriere, typischerweise einer Folie abgedeckt. Diese Keimbarriere stellt einerseits einen Schutz gegen eindringende Bakterien dar und regelt darüber hinaus auch den Gasaustausch mit der Umgebung. Über eine gezielte Einstellung der Wasserdampfdurchlässigkeit dieser Keimbarriere kann sichergestellt werden, dass unterhalb der Wundabdeckung ein genügend feuchtes Klima herrscht, ohne dass es zu einer Mazeration der Haut kommt. Zudem schützt diese Keimbarriere Kleidung und sonstiges Verbandsmaterial vor austretendem Wundexsudat.

Derzeit werden solche Keimbarrieren, z.B. Folien mit der Schaumstoffunterlage verklebt, indem entweder lösungsmittelbasierte Klebstoffe aufgebracht (typischerweise aufgesprüht) oder auch Heiss-Schmelzklebstoffe aufgetragen werden. Der Klebstoffauftrag kann hierbei entweder einseitig (typischerweise auf die Folie) oder beidseitig, also auf die Folie und den Schaum, erfolgen. Alternativ kann bspw. auch ein wärmeaktivierbares Klebevlies zwischen Folie und Schaumstoff eingelegt werden, wodurch der Verbund bewirkt wird. Es muss jedoch unter Anwendung eines Klebstoffs stets eine Zwischenschicht erzeugt werden, um die Verbindung zwischen Schaumstoff und Folie sicherzustellen.

Kritisch ist hierbei stets, dass die Klebeschicht den Transport von Wasserdampf nicht behindern darf; in der Regel sollte die Durchlässigkeit über 1000 g / m² / Tag liegen. Eine solche Durchlässigkeit kann bspw. über ein offenes Beschichtungsbild mit Klebstoff oder durch einen vollflächig aufgetragenen, jedoch wasserdampfdurchlässigen Klebstofffilm erreicht werden.

Die Gewährleistung der ausreichenden Wasserdampfdurchlässigkeit durch die Klebstoffschicht stellt in der Praxis während der Herstellung ein nicht zu unterschätzendes Problem dar, während die Durchlässigkeit durch die Folie und den Schaumstoff mit einfachen Routineversuchen ermittelt und eingestellt werden kann. Zudem verteuert die notwendige Klebstoffschicht die Herstellung insgesamt. Zudem ist die Verbindung von Folie und Schaumstoff durch eine Klebstoffschicht in der Praxis oftmals nicht überzeugend, da es immer wieder zu Ablösungen der Folie von dem Schaumstoff kommt.

Aus US 5,147,338 ist es bekannt, bei einer Wundabdeckung einen Polyurethan-Film auf einen Polyurethan-Schaumstoff aufzusprühen. Hierdurch kann die o.g. Klebstoffschicht entfallen. Das Aufsprühen eines Polyurethanfilms auf den Schaumstoff hat jedoch eine Vielzahl von Nachteilen: Erstens kann ein gleichmässiger Auftrag nur schwer gewährleistet werden. Zweitens penetriert das aufgesprühte Polyurethanmaterial signifikant in den Schaumstoff, was die Aufnahmefähigkeit des Schaumstoffs für Wundexsudat erheblich senken kann. Zudem müssen, um die Versprühbarkeit von Polyurethanmaterialien zu gewährleisten, Lösungsmittel zugesetzt werden; Lösungsmittelreste in der Wundabdeckung können jedoch nicht toleriert werden.

Aus US 3,668,050 ist ein Operationstuch mit einer Operationsöffnung bekannt. Das Operationstuch kann eine auf ein Schaumstoffmaterial extrudierte Folienschicht aufweisen. Das Schaumstoffmaterial ist auf der wundabgewandten Seite angeordnet und soll u.a. das Abrutschen von abgelegtem Operationsbesteck verhindern.

Aus EP 651 984 ist ein Pflaster bekannt mit einer äusseren, nicht porösen Folienschicht und einer dem Körper zugewandten Haftklebstoffschicht, in welche eine poröse Schicht z.B. aus Schaumstoff mit einer Dicke im Bereich von 0,01 bis 0,5 mm eingebettet ist. Der Haftklebstoff dringt hierbei bis zur äusseren Folienschicht durch; durch die Einbettung der porösen Schicht ist die Anbindung des Haftklebstoffs an das Pflaster fester.

Aus WO 95/15135 ist ein Wundabdeckung bekannt umfassend eine Kombination eine dampfdurchlässige Lage und eine diskontinuierliche Lage, wobei die dampfdurchlässige Lage über die Abmessungen der diskontinuierlichen Lage hinausgeht. Die Wundabdeckung soll u.a. eine vorteilhafte Absorption eines Wundexsudats aufweisen..

Es ist daher eine Aufgabe der vorliegenden Erfindung, die Nachteile des bekannten zu vermeiden, insbesondere eine Schaumstoff-Wundabdeckung und ein Herstellungsverfahren für eine Schaumstoff-Wundabdeckung bereitzustellen, welches in der Durchführung einfach und kostengünstig ist, keine Rückstände (z.B. Lösungsmittelreste) im fertigen Produkt enthält bzw. hinterlässt und im Ergebnis einen hervorragenden und dauerbelastbaren Verbund zwischen Schaumstoff und Folie sicherstellt, ohne dass die Aufnahmefähigkeit des Schaumstoffs für Wundexsudat herabgesetzt wird.

Diese Aufgabe wird durch eine Wundabdeckung und ein Verfahren zur Herstellung einer solchen Wundabdeckung gelöst, wie in den Ansprüchen definiert.

Das erfindungsgemässe Verfahren zur Herstellung einer mindestens zweischichtigen, im wesentlichen unterbruchsfrei flächigen Wundabdeckung, umfassend
- eine erste Schicht aus einem offenzelligem Schaumstoffmaterial, welche eine erste Hauptfläche und insbesondere eine zweite Hauptfläche aufweist; und
- eine zweite Schicht als wasserdampfdurchlässige Keimbarriere, insbesondere aus einem Folienmaterial, welche zweite Schicht unmittelbar an die erste Hauptfläche der ersten Schicht angrenzt,
umfasst die folgenden Schritte:
(a) Bereitstellen des Schaumstoffmaterials der ersten Schicht, insbesondere in einer Dicke zwischen 1 mm bis 10 mm, vorzugsweise zwischen 3 mm bis 5 mm;
(b) Auftragen, insbesondere Extrudieren mindestens eines thermoplastischen Materials auf eine Hauptfläche des Schaumstoffmaterials bei einer Temperatur oberhalb der Erweichungstemperatur des thermoplastischen Materials (2), welches Material anschliessend zur zweiten Schicht verfestigt.

Vorzugsweise wird die erste Schicht aus einem Schaumstoffmaterial hierbei nicht in eine Haftklebstoff-Schicht eingebettet, also im Gegensatz zu vorstehend genanntem Dokument EP 651 984.

Alternativ und/oder ergänzend ist es im Rahmen der Erfindung ebenfalls möglich, die erste Schicht aus einem thermoplastischen Schaumstoffmaterial auf eine Temperatur oberhalb der Erweichungstemperatur zu erhitzen. Thermoplastizität des Materials für die zweite Schicht ist dann nicht zwingend erforderlich. Nachteilig bei Verfahren unter Erwärmung des Schaumstoffmaterials ist jedoch, dass das Zusammenführen mit der zweiten Schicht dann unter Druck erfolgt, was bei nur ungenügend eingestellten Randbedingungen des Prozesses die Struktur des erwärmten Schaumstoffmaterials und damit ggf. die Fähigkeit zur Absorption von Wundexsudat und die Wasserdampfdurchlässigkeit beeinträchtigen könnte.

Unter den beiden "Hauptflächen" des Schaumstoffmaterials der ersten Schicht werden hierbei diejenigen Flächen verstanden, welche beim bestimmungsgemässen Gebrauch als Wundabdeckung im wesentlichen parallel zur abgedeckten Hautoberfläche des Verwundeten verlaufen, und zwar auf der wundabgewandten und der wundzugewandten Seite.

Als "im wesentlichen unterbruchsfrei" werden Flächen des erfindungsgemässen mehrschichtigen Materials verstanden, welche keine Öffnungen aufweisen, die der Etablierung des angestrebten feuchten Milieus unterhalb der Wundabdeckung bei bestimmungsgemässem Gebrauch signifikant zuwider laufen würden. Das Kriterium "im wesentlichen unterbruchsfrei" schliesst also nicht das Vorhandensein bspw. von geeignet dimensionierten Perforationslinien aus, entlang welcher ein Abtrennen von Teilstücken durch Abreissen ermöglicht ist.

Überraschenderweise hat sich herausgestellt, dass sich insbesondere durch das Extrudieren der Keimbarriere, vorzugsweise eines Folienmaterials direkt (also ohne Klebstoff-Zwischenschicht) auf das Schaumstoffmaterial Wundabdeckungen herstellen lassen, welche sowohl eine hervorragende Festigkeit des Verbunds als auch nur eine minimale Penetration des Keimbarrieren- bzw. Folienmaterials in den Schaumstoff aufweisen. Die Aufnahmefähigkeit des Schaumstoffs ist daher nicht negativ beeinflusst, wie es hingegen bei aufgesprühtem Folienmaterial beobachtet wird. Das Material zur Herstellung der Keimbarriere muss für die Extrusion (im Gegensatz zur Sprühtechnik) nicht gelöst vorliegen, wodurch keinerlei Rückstände von Lösungsmitteln in dem fertigen Produkt auftreten. Zudem ist die Herstellung einfach zu handhaben, die Schichtdicke des Auftrags kann mit herkömmlichen Extrusionsanlagen (insbesondere mit gängigen Breitschlitzdüsen) zuverlässig eingestellt und konstant gehalten werden. Bei geeignet gewählten Prozessbedingungen können alternativ oder ergänzend auch andere Verfahren als die Extrusion angewendet werden, bspw. Flammkaschierung, Kalandrierung, etc. Geeignete Verfahren sind dem Fachmann an sich geläufig. Im Rahmen der vorliegenden Erfindung ist jedenfalls darauf zu achten, dass die Prozessbedingungen derart gewählt werden, dass die vorstehend beschriebenen Anforderungen an die Aufnahmefähigkeit des Schaummaterials der ersten Schicht für das Wundexsudat, die Wasserdampfdurchlässigkeit beider Schichten und die Eigenschaft der zweiten Schicht als Keimbarriere nicht beeinträchtigt werden.

In weiteren bevorzugten Ausführungsformen der Erfindung wird als Schaumstoffmaterial ein Polyurethan-Schaumstoff bereitgestellt. Vorzugsweise ist das bereitgestellte Schaumstoffmaterial hydrophil. Besonders bevorzugt ist das Schaumstoffmaterial im wesentlichen offenzellig. Vorzugsweise kann eine durchschnittliche Porengrösse im Bereich von 0,02 mm bis 0,2 mm gewählt werden. Geeignete und besonders bevorzugte hydrophile, polyurethanbasierte Schaumstoffmaterialien sind dem Fachmann bekannt und im Markt erhältlich (bspw. Typ Vivo MCF 03 von Corpura B.V., 4879 NE Etten-Leur, The Netherlands; oder Typ 3014 von Polymer Health Technology, Ebbw Vale, NP23 8XE, United Kingdom).

In einer weiteren vorteilhaften Ausgestaltung der Erfindung wird das Schaumstoffmaterial in einer Dicke zwischen 1mm bis 10mm, vorzugsweise zwischen 3mm und 5mm, bereitgestellt. Derartige Dicken haben sich als hervorragender Kompromiss zwischen erforderlicher Aufnahmefähigkeit für Wundexsudat und Handhabbarkeit der Wundabdeckung erwiesen.

In besonders vorteilhaften Ausgestaltungen der Erfindung wird das mindestens eine Material in Schritt b) derart auf das Schaumstoffmaterial extrudiert, dass sich eine zweite Schicht mit einer Dicke zwischen 15 µm bis 100 µm, vorzugsweise 20 µm bis 40 µm, ausbildet. Dicken in den angegebenen Bereichen beeinflussen die Handhabbarkeit der Wundabdeckung wenig bis gar nicht, und das o.g. Steuern der Wasserdampfdurchlässigkeit der resultierenden Folie kann in diesem Dickenbereich an alle in der Praxis auftretenden Anforderungen angepasst werden.

Es wurde als besonders vorteilhaft gefunden, dass die Extrusion des mindestens einen Materials in Schritt b) bei einer Temperatur im Bereich zwischen 150 °C und 240 °C, vorzugsweise zwischen 180 °C und 220 °C, insbesondere zwischen 200 °C und 210 °C erfolgt.

In einer weiteren, besonders bevorzugten Ausführungsform können in Schritt b) zwei Schichten erzeugt werden, insbesondere durch Extrusion zweier Materialien, entweder sequentiell oder mittels Co-Extrusion. Auch das Aufbringen einer ersten Folienschicht mittels Extrusion und einer weiteren Schicht bspw. mittels Sprühen ist möglich. Insbesondere ist es durch die Erfindung ermöglicht, bspw. eine dünne, dem Schaumstoff zugewandte Schicht zu erzeugen, welche optimiert ist für die Haftung auf dem Schaumstoff; Schichtdicken von 5 µm bis 10 µm haben sich hierfür bereits als ausreichend erwiesen. Anschliessend kann als aussenliegende, zweite Schicht noch eine Schicht mit z.B. höherer mechanischer Festigkeit (typischerweise in einer Dicke von etwa 10 µm bis 20 µm) aufgetragen, oder gleichzeitig coextrudiert werden. Auf diese Weise können also bspw. Folienschichten erzeugt werden, welche aus mindestens zwei Teil-Schichten aufgebaut sind, wobei die Folienschicht insgesamt sehr dünn sein kann, jedoch trotzdem eine sehr gute Haftung auf dem Schaumstoffmaterial und ein weiches Griffbild aufweist. Zudem ist es durch die Verwendung zweier unterschiedlicher Schichten auch möglich, die Wasserdampfdurchlässigkeit über die Materialauswahl der zusätzlichen Schicht zu steuern, unabhängig von der Gesamtdicke der Folienschicht.

In einer weiteren Ausführungsform der Erfindung kann das mindestens eine Material während und/oder nach dem Aufbringen, insbesondere der Extrusion in Schritt b) aufgeschäumt werden. Die Aufschäumung eines Materials während und/oder nach der Extrusion ist dem Fachmann an sich geläufig und kann mit konventionellen Mitteln (bspw. durch den Zusatz eines Treibmittels wie Azodicarbonamid oder durch nachträgliche Wärmeeinwirkung) erfolgen. Vorteilhafterweise könnte bspw. eine erste, aufgeschäumte Schicht auf den Schaumstoff extrudiert werden, mit einer geringeren Porengrösse als die darunter liegende Schaumstoffschicht. Anschliessend (oder auch gleichzeitig durch Coextrusion) kann dann die abschliessende Schicht, z.B. eine Folienschicht aufgebracht werden. Durch die somit nicht abrupte, sondern stufenweise Abnahme der Porosität und/oder Übergang von Offen- zu Geschlossenzelligkeit kann eine weitere Verbesserung der Haftung zwischen wundzugewandtem Schaumstoff und aussenliegender Folie erzielt werden, wobei zudem noch eine üblicherweise gewünschte, optisch glattere wundabgewandte Seite resultiert.

Selbstverständlich kann auf der ersten, wundseitigen Schicht aus einem Schaumstoffmaterial insbesondere auch durch Extrusion eine zweite Schicht als Keimbarriere aus einem Schaumstoffmaterial erzeugt werden, z.B. auch ohne zusätzliche, aussenliegende (Folien)Schicht(en). Falls als Keimbarriere also ein Schaumstoff zur Anwendung kommt, so muss es sich hierbei um einen geeigneten, im wesentlichen geschlossenzelligen Schaumstoff handeln, um die Wirkung als Keimbarriere und insbesondere auch die Etablierung des angestrebten feuchten Milieus unterhalb der Wundabdeckung zu gewährleisten. Die Erzeugung eines geschlossenzelligen Schaumstoffs durch und/oder nach der Extrusion ist dem Fachmann geläufig, wie vorstehend dargelegt. Ggf. anzupassende Randbedingungen der Extrusion und/oder der Wärmebehandlung können vom Fachmann leicht in Routineversuchen ermittelt werden.

Besonders bevorzugt wird nach der Extrusion des Materials in Schritt b) das Extrudat mittels einer gekühlten Walze an die erste Schicht bzw. den Schaumstoff angepresst. Hierbei findet jedoch keine bzw. nur eine minimale Penetration des Schaumstoffs statt, weil das Extrudat vorgeformt auf den Schaumstoff auftrifft und insbesondere die Viskosität der Schmelze bereits möglichst hoch ist.

Es ist weiter bevorzugt, dass in Schritt b) ein thermoplastisches Polyurethanmaterial, insbesondere ein Polyetherpolyurethan, bereitgestellt wird. Thermoplastische Polyetherpolyurethane mit Zulassung für den medizinischen Bereich sind dem Fachmann bekannt und im Markt erhältlich. Besonders geeignete thermoplastische Materialien weisen gemäss ISO 1133 einen Melt Flow Index (MFI) zwischen 5 und 50 g / 10 min bei 170 °C und einem Stempelgewicht von 21.6 kg auf.

Ein weiterer Aspekt der Erfindung betrifft eineft zweischichtige flächige Wundabdeckung erhältlich durch eines der vorgenannten Verfahren, umfassend eine erste Schicht aus einem offenzelligem Schaumstoffmaterial, und eine zweite Schicht als wasserdampfdurchlässige Keimbarriere, insbesondere aus einem Folienmaterial, welche zweite Schicht unmittelbar an eine Hauptfläche der ersten Schicht angrenzt, wobei bei einer Porengrösse des im wesentlichen offenzelligen Schaumstoffmaterials im Bereich von 0,02 mm bis 0,2 mm das Material der zweiten Schicht nicht mehr als 0,01 mm in das Schaumstoffmaterial eingedrungen ist. Eine derartige minimale Eindringtiefe lässt sich insbesondere durch ein Verfahren wie vorstehend im Detail dargestellt bewirken, wohingegen bei konventionellem Sprühauftrag von Polyurethan-Lösungen eine Eindringtiefe von mindestens etwa 0,05 mm resultiert.

Ein zusätzlicher Aspekt der Erfindung betrifft die Verwendung eines Extrusionsverfahrens in der Herstellung einer Wundabdeckung zum Aufbringen einer Wasserdampf durchlässigen Keimbarriere, insbesondere einer Folienschicht, unmittelbar auf eine offenzellige Schaumstoffschicht.

Die Erfindung wird nachfolgend anhand von Ausführungsbeispielen und Figuren erläutert, ohne dass der Gegenstand der Erfindung auf diese Ausführungsbeispiele zu beschränken wäre. Es zeigen:
- Fig. 1:: Wundabdeckung mit Schaumstoffschicht und aussenliegender Folie;
- Fig. 2:: Wundabdeckung mit Schaumstoffschicht und zwei aussenliegenden Folienschichten;
- Fig. 3:: Herstellungsverfahren, schematisch;
- Fig. 4:: Wundabdeckung auf einem Arm.

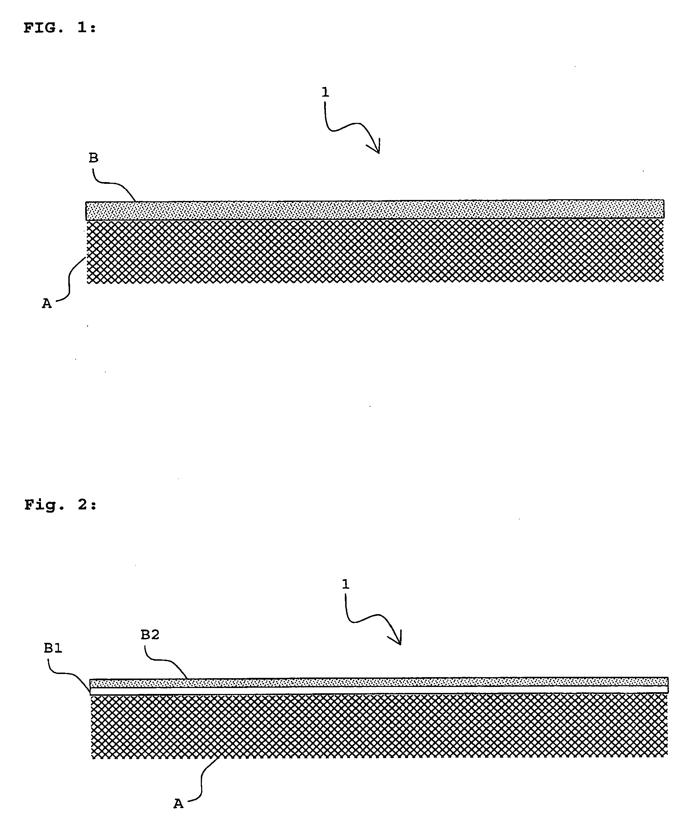

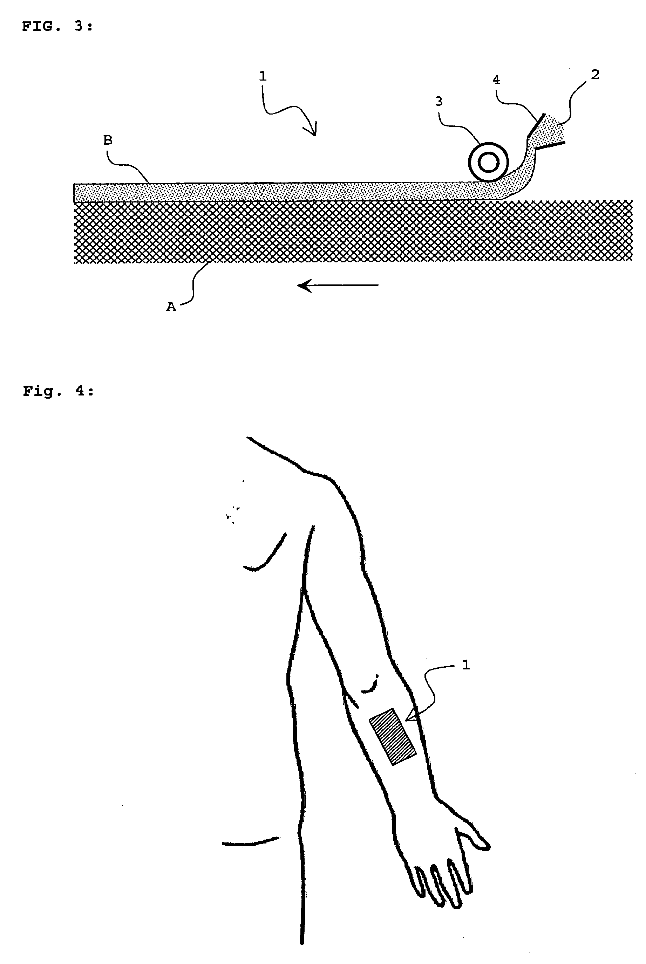

Fig. 1 zeigt schematisch vereinfacht eine erfindungsgemässe Wundabdeckung 1, erhältlich mit dem vorstehend im Detail beschriebenen und in Fig. 3 illustrierten Verfahren, umfassend eine der Wunde zugewandte erste Schicht A aus einem Schaumstoffmaterial, und eine zweite Schicht B als Keimbarriere aus einem Folienmaterial. Die beiden Schichten A und B sind ohne eine Zwischenschicht verbunden; insbesondere liegt keine Klebstoffschicht zwischen der ersten Schicht A und der zweiten Schicht B. Besonders zuverlässige Verbindungen werden erhalten, wenn die Materialien von erster Schicht A und zweiter Schicht B chemisch verwandt sind. Vorteilhaft handelt es sich bei der ersten Schicht A um einen hydrophilen Polyurethanschaumstoff, und bei Schicht B um eine Schicht aus Polyetherpolyurethan; aufgrund der ähnlichen chemischen Natur der beiden Schichten lässt sich eine besonders stabile Verbindung erzielen.

Fig. 2 zeigt eine Ausführungsform einer erfindungsgemässen Wundabdeckung, bei welcher auf einer ersten Schicht A aus einem Schaumstoffmaterial eine zweite Schicht aufgetragen ist, welche die Teilschichten B1 und B2 umfasst. Die Schichten B1 und B2 können entweder gemeinsam, bspw. mittels Coextrusion (oder sequentieller Extrusion) erzeugt werden. Auch das Erzeugen nur der Schicht B1 mittels Extrusion ist möglich, wobei die Schicht B2 z.B. anschliessend aufgesprüht wird. Die Schicht B2 kann, muss jedoch nicht flächig aufgetragen sein; insbesondere kann es sich hierbei auch um einen Aufdruck handeln, umfassend Schriftzeichen wie Herstellerangaben, Markennamen oder auch Schnittlinien, für die Erleichterung eines passgenauen Zuschnitts der Wundabdeckung vor Ort.

Fig. 3 illustriert schematisch eine Ausführungsform des erfindungsgemässen Herstellungsverfahrens anhand einer Wundabdeckung. Eine erste Schicht A eines Schaumstoffmaterials wird in einer herkömmlichen, geeigneten Extrusionsanlage (nicht im Detail gezeigt) entlang der Pfeilrichtung gefördert. Um eine Förderung des Schaumstoffmaterials zuverlässig zu gewährleisten, kann ggf. ein steifes Trägermaterial für die Schicht A vorgesehen werden. Über eine Düse 4, typischerweise eine Breitschlitzdüse, wird ein thermoplastisches Material (hier: ein thermoplastisches Polyetherpolyurethan) extrudiert, typischerweise bei einer Temperatur zwischen 150 °C und 240 °C, vorzugsweise bei 180°C und 220°C. Die Düse 4 hat hierbei keinen direkten Kontakt mit der Schicht A, sondern dass Material 2 verlässt die Düse vorgeformt und wird gewissermassen auf der Schicht A abgelegt und anschliessend vorzugsweise mit einer gekühlten Walze 3 derart an die Schicht A angedrückt, so dass eine feste Verbindung zwischen Schicht A und der aus dem Material 2 gebildeten Schicht B gebildet wird.

Fig. 4 zeigt eine Wundabdeckung 1 gemäss der Erfindung im Gebrauch, hier exemplarisch als Abdeckung einer Wunde an einem Arm. In dieser Draufsicht ist die wundabgewandte Schicht B schraffiert dargestellt. Auf der wundzugewandten Seite befindet sich (in der Figur) verdeckt die Schicht A aus einem Schaumstoffmaterial. Eine Fixierung der Wundabdeckung ist bspw. mit Haftklebestreifen möglich. Die Grösse der Wundabeckung 1 ist an die jeweilige Wundgrösse angepasst zu wählen, entweder durch Verwendung vorkonfektionierter Wundabdeckungen oder durch Abtrennung (bspw. durch Abreissen entlang von Perforierungen), insbesondere Zuschnitt (bspw. entlang vorzugsweise auf die Schicht A aufgedruckter Markierungen) einer geeignet grosser Wundabdeckung aus einer grösseren Einheit.

Mit dem erfindungsgemässen Verfahren erhaltene Wundverbände wurden auf die Festigkeit des Verbunds zwischen Folie und Schaumstoff analysiert und mit einem derzeit handelsüblichen Produkt verglichen. Als Material für die Herstellung der Folienschicht (Schicht B) wurde Perlathane^{®} D16N85 (Hersteller Merquinsa), MFI 10 g / 10 min bei 170 °C (Stempelgewicht 21.6 kg), Extrusionstemperatur 205 °C verwendet; die jeweils verwendete Schaumstoffunterlage (Schicht A) ist in Tabelle 1 angegeben. Hierbei wurden die folgenden Testmethoden verwendet:

### Testmethode 1: Haftung

Auf die Polyurethan-Schaumseite von 100 cm² (10 x 10 cm Muster) des Verbunds aus Schicht A und Schicht B werden 5ml einer wässrigen 0.9 % NaCl-Lösung gegeben. Die befeuchteten Muster werden anschliessend bei 40°C in einer gesättigten Wasserdampfatmosphäre während 24 h gelagert. Nach der Entnahme der Prüflinge werden diese während 10 Minuten auf Raumtemperatur abgekühlt. Anschliessend wird die Haftung zwischen Polyurethan-Folie und Polyurethan-Schaum von Hand qualitativ beurteilt. Zum Vergleich werden Proben beurteilt, welche nicht feucht und warm gelagert wurden. Die gefundene Haftung wird nach einem Notensystem (1 = unbrauchbar, 2 = schwach, 3 = ungenügend, 4 = genügend, 5 = gut, 6 = ausgezeichnet) klassifiziert.

### Testmethode 2: Dichtigkeit

Auf die Polyurethan-Schaumseite von 100 cm² (10 x 10 cm Muster) des Verbunds aus Schicht A und Schicht B werden 5 ml einer wässrigen 0.9 % NaCl-Lösung, welche mit 0.1 % Methylenblau eingefärbt ist, gegeben. Nach 1 Stunde (Raumtemperatur) wird die Dichtigkeit auf der Polyurethan-Folienseite (Schicht B) optisch beurteilt. Die gefundene Dichtigkeit wird nach einem Notensystem (1 = unbrauchbar, 2 = schwach, 3 = ungenügend, 4 = genügend, 5 = gut, 6 = ausgezeichnet) klassifiziert.

Die erhaltenen Resultate sind in Tabelle 1 angegeben:

**Tabelle 1: Beurteilung von Haftung und Dichtigkeit erfindungsgemässer Wundverbände.**

| PUR-Film extrudiert auf: | Vivo MCF 03 (Corpura B.V.) | Foam 3014 (Polymer Health Technology) | **Referenzmuster** 3M Foam Dressing (Art. 90601) |
|---|---|---|---|
| Haftung zwischen Schaum und Film (Blindwert) | 6 | 6 | 5 |
| Haftung zwischen Schaum und Film nach 24h Feuchtlagerung bei 40°C | 5 | 5 | 3-4 |
| Dichtigkeit nach 1 Stunde | 6 | 6 | 6 |

Es ist ersichtlich, dass die Festigkeit des Verbunds zwischen Schicht A (Schaumstoff) und Schicht B (Folie) bei den erfindungsgemässen Wundverbänden bzw. durch das erfindungsgemässe Herstellungsverfahren für einen solchen Wundverband unter typischer Praxisbeanspruchung verbessert ist.

## Patentansprüche

1. Verfahren zur Herstellung einer mindestens zweischichtigen, im wesentlichen unterbruchsfrei flächigen Wundabdeckung (1), umfassend
- eine erste Schicht (A) aus einem offenzelligen Schaumstoffmaterial, aufweisend eine erste Hauptfläche und eine zweite Hauptfläche; und
- eine zweite Schicht (B) als wasserdampfdurchlässige Keimbarriere, insbesondere aus einem Folienmaterial, welche zweite Schicht (B) unmittelbar an die erste Hauptfläche der ersten Schicht (A) angrenzt,
umfassend die folgenden Schritte:
(a) Bereitstellen des Schaumstoffmaterials der ersten Schicht (A), insbesondere in einer Dicke zwischen 1 mm bis 10 mm, vorzugsweise zwischen 3 mm bis 5 mm;
(b) Flächiges Auftragen, insbesondere Extrudieren mindestens eines thermoplastischen Materials (2) auf die Hauptfläche des Schaumstoffmaterials (A) bei einer Temperatur oberhalb der Erweichungstemperatur des thermoplastischen Materials (2), und Verfestigen des Materials (2) zur zweiten Schicht (B);
wobei die erste Schicht aus einem Schaumstoffmaterial nicht in eine Haftklebstoff-Schicht eingebettet wird.

2. Verfahren gemäss Anspruch 1,
**dadurch gekennzeichnet, dass** als Schaumstoffmaterial der ersten Schicht (A) ein Polyurethan-Schaumstoff bereitgestellt wird.

3. Verfahren gemäss einem der Ansprüche 1 oder 2,
**dadurch gekennzeichnet, dass** die erste Schicht (A) hydrophil ist.

4. Verfahren gemäss einem der Ansprüche 1 bis 3,
**dadurch gekennzeichnet, dass** das mindestens eine Material (2) in Schritt b) derart auf das Schaumstoffmaterial extrudiert wird, dass sich eine zweite Schicht (B) mit einer Dicke zwischen 15 µm bis 100 µm, vorzugsweise zwischen 20 µm bis 40 µm ausbildet.

5. Verfahren gemäss einem der Ansprüche 1 bis 4,
**dadurch gekennzeichnet, dass** die Extrusion des mindestens einen Materials (2) in Schritt b) bei einer Temperatur im Bereich zwischen 150 °C und 240 °C, vorzugsweise zwischen 180 °C und 220 °C, insbesondere zwischen 200 °C und 210 °C erfolgt.

6. Verfahren gemäss einem der Ansprüche 1 bis 5,
**dadurch gekennzeichnet, dass** in Schritt b) zwei Schichten (B1,B2) erzeugt werden, insbesondere durch Extrusion zweier Materialien, entweder sequentiell oder mittels Co-Extrusion.

7. Verfahren gemäss einem der Ansprüche 1 bis 6,
**dadurch gekennzeichnet, dass** das mindestens eine Material (2) während und/oder nach der Extrusion in Schritt b) aufgeschäumt wird.

8. Verfahren gemäss einem der Ansprüche 1 bis 7,
**dadurch gekennzeichnet, dass** nach der Extrusion des Materials (2) in Schritt b) das Extrudat mittels einer gekühlten Walze (3) an die erste Schicht (A) angepresst wird.

9. Verfahren gemäss einem der Ansprüche 1 bis 8,
**dadurch gekennzeichnet, dass** als Material (2) in Schritt b) ein thermoplastisches Polyurethanmaterial, insbesondere ein Polyetherpolyurethan, bereitgestellt wird.

10. Mindestens zweischichtige, im wesentlichen unterbruchsfrei flächige Wundabdeckung (1), erhältlich durch ein Verfahren gemäss einem der Ansprüche 1 bis 9, umfassend eine erste Schicht (A) aus einem offenzelligen Schaumstoffmaterial insbesondere in einer Dicke zwischen 1 mm bis 10 mm, vorzugsweise zwischen 3 mm bis 5 mm, und eine zweite Schicht (B) als wasserdampfdurchlässige Keimbarriere, insbesondere aus einem Folienmaterial, welche zweite Schicht unmittelbar an eine Hauptfläche der ersten Schicht (A) angrenzt, **dadurch gekennzeichnet, dass**, insbesondere bei einer Porengrösse des im wesentlichen offenzelligen Schaumstoffmaterials im Bereich von 0,02 mm bis 0,2 mm, das Material der zweiten Schicht (B) nicht mehr als 0,01 mm in das Schaumstoffmaterial eingedrungen ist.

11. Verwendung eines Extrusionsverfahrens in der Herstellung einer Wundabdeckung zum Aufbringen einer wasserdampfdurchlässigen Keimbarriere, insbesondere einer Folienschicht, unmittelbar auf eine offenzellige Schaumstoffschicht.

## Claims

1. Method for producing an at least two-layered, substantially uninterruptedly flat wound cover (1), comprising
- a first layer (A) of an open-celled foam material, having a first main area and a second main area; and
- a second layer (B) as water vapor-permeable germ barrier, in particular of a film material, which second layer (B) is directly adjacent to the first main area of the first layer (A),
comprising the following steps:
(a) providing the foam material of the first layer (A), in particular in a thickness between 1 mm to 10 mm, preferably between 3 mm to 5 mm;
(b) flat application, in particular extrusion, of at least one thermoplastic material (2) onto the main area of the foam material (A) at a temperature above the softening temperature of the thermoplastic material (2), and solidifying the material (2) to form the second layer (B);
the first layer of a foam material not being imbedded into a pressure-sensitive adhesive layer.

2. Method according to Claim 1, **characterized in that** a polyurethane foam is provided as foam material of the first layer (A).

3. Method according to either of Claims 1 and 2, **characterized in that** the first layer (A) is hydrophilic.

4. Method according to any one of Claims 1 to 3, **characterized in that** the at least one material (2) in step b) is extruded onto the foam material in such a way as to form a second layer (B) having a thickness between 15 µm to 100 µm, preferably between 20 µm to 40 µm.

5. Method according to any one of Claims 1 to 4, **characterized in that** the extrusion of the at least one material (2) in step b) takes place at a temperature in the range between 150°C and 240°C, preferably between 180°C and 220°C, in particular between 200°C and 210°C.

6. Method according to any one of Claims 1 to 5, **characterized in that** two layers (B1,B2) are produced in step b), in particular by extrusion of two materials, either sequentially or by means of co-extrusion.

7. Method according to any one of Claims 1 to 6, **characterized in that** the at least one material (2) is foamed during and/or after extrusion in step b).

8. Method according to any one of Claims 1 to 7, **characterized in that**, after the extrusion of the material (2) in step b), the extrudate is pressed onto the first layer (A) by means of a cooled roll (3).

9. Method according to any one of Claims 1 to 8, **characterized in that** a thermoplastic polyurethane material, in particular a polyether polyurethane, is provided as material (2) in step b).

10. At least two-layered, substantially uninterruptedly flat wound cover (1), obtainable by a method according to any one of Claims 1 to 9, comprising a first layer (A) of an open-celled foam material in particular in a thickness between 1 mm to 10 mm, preferably between 3 mm to 5 mm, and a second layer (B) as water vapor-permeable germ barrier, in particular of a film material, which second layer is directly adjacent to a main area of the first layer (A), **characterized in that**, in particular at a pore size of the substantially open-celled foam material in the range from 0,02 mm to 0,2 mm, the material of the second layer (B) has penetrated not more than 0,01 mm into the foam material.

11. Use of an extrusion process in the production of a wound cover for applying a water vapor-permeable germ barrier, in particular a film layer, directly to an open-celled foam layer.

## Revendications

1. Procédé de fabrication d'un pansement pour plaies plat, au moins bicouche, essentiellement exempt d'interruptions (1), comprenant
- une première couche (A) en un matériau moussé à cellules ouvertes, comprenant une première surface principale et une seconde surface principale ; et
- une seconde couche (B) en tant que barrière contre les germes, perméable à la vapeur d'eau, notamment en un matériau en feuille, ladite seconde couche (B) étant directement adjacente à la première surface principale de la première couche (A),
comprenant les étapes suivantes :
(a) la mise à disposition du matériau moussé de la première couche (A), notamment en une épaisseur comprise entre 1 mm et 10 mm, de préférence entre 3 mm et 5 mm;
(b) l'application à plat, notamment l'extrusion, d'au moins un matériau thermoplastique (2) sur la surface principale du matériau moussé (A) à une température supérieure à la température de ramollissement du matériau thermoplastique (2), et la consolidation du matériau (2) pour former la seconde couche (2);
la première couche en un matériau moussé n'étant pas incorporée dans une couche adhésive sensible à la pression.

2. Procédé selon la revendication 1, **caractérisé en ce qu'**une mousse de polyuréthane est mise à disposition en tant que matériau moussé de la première couche (A).

3. Procédé selon l'une quelconque des revendications 1 ou 2, **caractérisé en ce que** la première couche (A) est hydrophile.

4. Procédé selon l'une quelconque des revendications 1 à 3, **caractérisé en ce que** le ou les matériaux (2) sont extrudés à l'étape b) sur le matériau moussé de manière à former une seconde couche (B) d'une épaisseur comprise entre 15 µm et 100 µm, de préférence entre 20 µm et 40 µm.

5. Procédé selon l'une quelconque des revendications 1 à 4, **caractérisé en ce que** l'extrusion du ou des matériaux (2) à l'étape b) a lieu à une température dans la plage comprise entre 150 °C et 240 °C, de préférence entre 180 °C et 220 °C, notamment entre 200 °C et 210 °C.

6. Procédé selon l'une quelconque des revendications 1 à 5, **caractérisé en ce que** deux couches (B1, B2) sont formées à l'étape b), notamment par extrusion de deux matériaux, soit séquentiellement, soit par co-extrusion.

7. Procédé selon l'une quelconque des revendications 1 à 6, **caractérisé en ce que** le ou les matériaux (2) sont moussés pendant et/ou après l'extrusion à l'étape b).

8. Procédé selon l'une quelconque des revendications 1 à 7, **caractérisé en ce qu'**après l'extrusion du matériau (2) à l'étape b), l'extrudat est comprimé par un cylindre refroidi (3) sur la première couche (A).

9. Procédé selon l'une quelconque des revendications 1 à 8, **caractérisé en ce qu'**un matériau de polyuréthane thermoplastique, notamment un polyéther-polyuréthane, est mis à disposition en tant que matériau (2) à l'étape b).

10. Pansement pour plaies plat, au moins bicouche, essentiellement exempt d'interruptions (1), pouvant être obtenu par un procédé selon l'une quelconque des revendications 1 à 9, comprenant une première couche (A) en un matériau moussé à cellules ouvertes, notamment d'une épaisseur comprise entre 1 mm et 10 mm, de préférence entre 3 mm et 5 mm, et une seconde couche (B) en tant que barrière contre les germes, perméable à la vapeur d'eau, notamment en un matériau en feuille, ladite seconde couche étant directement adjacente à une surface principale de la première couche (A), **caractérisé en ce que**, notamment à une taille de pore du matériau moussé essentiellement à cellules ouvertes dans la plage allant de 0,02 mm à 0,2 mm, le matériau de la seconde couche (B) ne pénètre pas à plus de 0,01 mm dans le matériau moussé.

11. Utilisation d'un procédé d'extrusion pour la fabrication d'un pansement pour plaies, pour l'application d'une barrière contre les germes, perméable à la vapeur d'eau, notamment d'une couche en feuille, directement sur une couche de mousse à cellules ouvertes.
